# EUROPEAN PATENT APPLICATION

(11) **EP 2 460 470 A1**
(43) Date of publication of application: **06.06.2012**
(21) Application number: 10804440.5
(22) Date of filing: 28.07.2010
(51) Int. Cl.: A61B 5/1455

(54) **DEVICE FOR ESTIMATING BLOOD SUGAR LEVEL**

(30) Priority: 28.07.2009 JP 2009175863
(71) Applicant: Panasonic Electric Works Co., Ltd., Kadoma-shi Osaka 571-8686 (JP)
(72) Inventor: MARUO, Katsuhiko, Kadoma-shi Osaka 571-8686 (JP)
(74) Representative: Appelt, Christian W.
(86) International application number: PCT/JP2010/062680
(87) International publication number: WO 2011/013694

(57) **Abstract**

The blood sugar value estimation apparatus is configured to non-invasively calculate the blood sugar value with time on the basis of the optical spectrum of the living body measured with time and the calibration model. The apparatus comprises a calibration model creating means configured to create a calibration model from the calibration models or a calibration model from a plurality of the datasets for creating the calibration model. The apparatus is configured to measure a bio-spectrum of a person being tested to set the reference spectrum, and to measure a difference spectrum of a difference between the reference spectrum and a measurement spectrum measured at a time other than a time when the reference spectrum is measured, and to change the calibration model for calculating according to the variation of the difference spectrum. Consequently, the blood sugar value is estimated, especially monitored with time, with high-accuracy.

## Description

### TECHNICAL FIELD

This invention relates to blood sugar value estimation apparatus using near infrared rays. Particularly, this invention relates to a blood sugar value estimation apparatus adapted to be preferably used for monitoring, with time, the blood sugar value.

### BACKGROUND ART

The method of applying the near infrared ray to the living tissue to measure the diffusely reflected light which is diffused in the living tissue or to measure the transmitted light which is transmitted through the living tissue is made. On the basis of the signal or the spectrum obtained from the diffusely reflected light or the transmitted light, the qualitatively analyzing and quantitatively analyzing the biogenic substance and behavior is made. Such the analysis makes it possible to instantaneously and non-invasively obtain the various information of the living body without test reagent. Therefore, in the medical field, the above mentioned method is widely used for the measurement of the amount of oxygen in the blood.

Such the application of the concentration of constituents of the living body with respect to the measurement of the blood sugar value is highly required in view of the blood sugar value management of the diabetic patient, conventionally. In addition, recently, management of the blood sugar value within adequate range in intensive-care unit (ICU) is verified for improvement of the medical effect. Therefore, the application of the concentration of constituents of the living body with respect to the measurement of the blood sugar value is expected to be applied to the medical treatment.

The methods of measuring the blood sugar value from the near infrared ray obtained from the living tissue are numerously proposed, and are for example disclosed in Japanese patent application publication No. 2006-87913A. Fig. 2 shows a blood sugar value estimation apparatus of non-invasive type of the above publication. In the blood sugar value estimation apparatus, the near infrared ray emitted from the halogen lamp 1 is entered into the living tissue 6 through the heat shield plate 2, the pin hole 3, the lens 4, and the light fiber bundle 5. The light fiber bundle 5 comprises a measurement light fiber 7 and a reference light fiber 8. The measurement light fiber 7 is connected at its tip to the measurement probe 9. The light fiber 8 for reference is connected at its tip to the reference probe 10. The reference probe 10 is opposed to the reference plate 18. Furthermore, the measurement probe 9 and the reference probe 10 are connected to the measurement output unit 11 and the reference output unit 12, respectively.

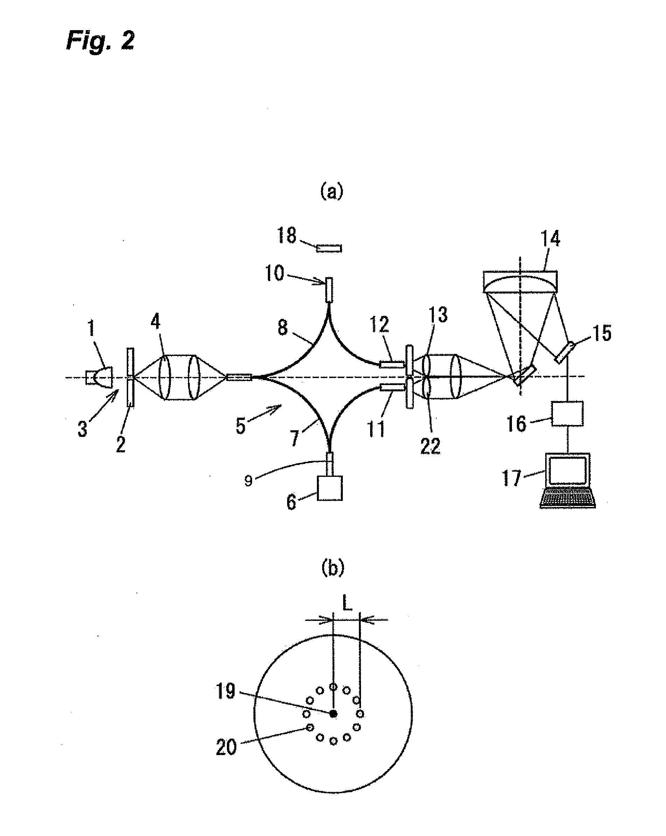

Each one of the measurement probe 9 and the reference probe 10 has an end face shown in Fig. 2 (b), and is provided at its end face with one light receiving fiber 19 and a plurality of the light emitting fibers 20. The light receiving fiber 19 is located at a center of the end face, and the light emitting fibers 20 are located on the circumference of circle. The light emitting fibers 20 and the light receiving fiber 19 are arranged such that the center-to-center distance of the light emitting fiber 20 and the light receiving fiber 19 is, for example, set to be 0.65 millimeters.

When the measurement of the near infrared spectrum is performed under a condition where the end surface of the measurement probe 9 is contacted to the surface of the living tissue 6 such as the forearm of the living body with a predetermined pressure, the near infrared ray is entered into the light fiber bundle 5 from the light source 1, transmitted through the measurement light fiber 7, and finally, is applied to the surface of the living tissue 6 from the light fibers 20 arranged on the circumference of the circle of the end face of the measurement probe 9.

The measurement light applied to the living tissue 6 is diffusely reflected in the living tissue, and then a part of the diffusely reflected light is received by the light receiving fiber 19 on the end face of the measurement probe 9. The received light is output to the measurement output member 11 through the light receiving fiber 19. The received light is entered to the diffraction grating through the lens 13, whereby the light is dispersed. After dispersing, the light is detected by the light receiving element 15. The light signal from the light receiving element 15 is made analog-digital conversion by the A/D converter 16. Then, the result is input to the computing unit 17 such as personal computer. The computing unit 17 analyzes the obtained spectrum data to calculate the blood sugar value. The reference numeral 22 in the illustration is a shutter.

In the reference measurement, the light reflected by the reference plate 18 such as ceramic plate is measured. The measured light is defined as the reference light. That is, when the near infrared ray is entered into the light fiber bundle 5 from the light source 1, the near infrared ray passes through the reference light fiber 8, and applied to the surface of the reference plate from the end of the reference probe 10. The light applied to the reference plate 18 is output from the reference output unit 12 through the light receiving fiber 19 in the end of the reference probe 10. The shutter 22 is disposed between the measurement output unit 11 and the lens 13, and between the reference output unit 12 and the lens 13. According to the opening and closing of the shutter 22, the light passes through either the measurement output unit 11 or the reference output unit 12, selectively.

The reasons of the value of the center-to-center distance L between the light emitting fiber 20 and the light receiving fiber 19 being set to the above is to selectively measure the spectrum in the inner skin from the skin tissue having the layer structure including the surface skin, the inner skin, and the subcutaneous tissue.

By the way, when the blood sugar value is measured quantitatively, the signal indicative of the glucose concentration in the target living tissue is very small. Therefore, the accuracy of the quantitative determination depends on the adequacy-and-inadequacy of calibration model (calibration curve) for calculating the blood sugar value from the measured spectrum greatly.

In view of selecting suitable calibration model, Japanese patent publication No. 3931638B discloses the method of estimating the skin thickness from the form of the near infrared spectrum, and using the calibration model for quantitative estimation according to the estimated skin thickness of the skin tissue from the calibration models which are prepared in advance.

In addition, in order to create the calibration model having a high estimation accuracy, a plurality of the data are required. The availability of the simulation with respect to the above is well known such as Japanese patent application publication No. 2006-87913A. The patent application publication discloses the method of creating the calibration model by calculating the difference absorption spectrum of the difference between the absorption spectrum obtained by the simulation and the reference absorption spectrum, calculating the synthesized absorption spectrum by synthesizing the difference absorption spectrum with the absorption spectrum measured from the person being tested, and multiply analyzing the synthesized absorption spectrum.

### PRIOR ART

PATENT LITERATURE 1: Japanese patent application publication No. 2006-87913 A
PATENT LITERATURE 2: Japanese patent application publication No. 2004-138454 A
PATENT LITERATURE 3: Japanese patent publication No. 3931638

### NONPATENT LITERATURE

NON-PATENT LITERATURE 1: T.L. Troy and S.N. Thennadil, J. Biomedical Optics, 6, 167 (2001)
NON-PATENT LITERATURE 2: C.R. Simpson, M. Kohl, M. Essenpreis, M. Cope, Phys. Med. Biol., 43, 2465 (1998)

### DISCLOSURE OF THE INVENTION

### PROBLEM TO BE SOLVED BY THE INVENTION

In the conventional near infrared ray spectroscopy, a plurality of the spectrums having numeral variations are collected when creating the calibration model. On the basis of the spectrums, the calibration model is created. This method is effective to achieve the robust quantitative determination. However, this method is realized when the sufficient SN ratio with respect to the target component to be made quantitative determination is ensured. When the components of the living body, especially, the blood sugar value which is minor component, is made quantitative determination, it is preferred to use the calibration model which is capable of considering the disturbance caused in the period when the measurement is performed, compared with using the calibration model which is capable of considering various disturbances. It is possible to obtain the accurate result realistically when the calibration model which is capable of considering the disturbance caused in the period when the measurement is performed is used.

In addition, when such the calibration model is used, it is important for the accurate measurement to anticipate the disturbance in the period of being measured and to determine the selecting method of selecting the calibration model to be applied.

Furthermore, when the calibration model is synthesized by combining the spectrum on the basis of the numerical simulation, how to set the disturbance is important for improvement of the measurement accuracy. In addition, in order to measure accurately, there is a need to use the spectrum with combination of the disturbance caused in the measurement period. In addition, needless to say, it is preferred that the disturbance is to be easily set.

This invention is achieved in view of the above. An objective of this invention is to produce the blood sugar value estimation apparatus having a high-accurate estimation for monitoring the blood sugar value of the minor component, especially with time.

### MEANS OF SOLVING THE PROBLEM

This invention is a blood sugar value estimation apparatus for non-invasively calculate a blood sugar value with time on the basis of "an optical spectrum, with time, of a living body" and "a calibration model". The blood sugar value estimation apparatus comprises a calibration model creating means which is configured to create the calibration model from a plurality of the calibration models, or which is configured to create the calibration model from a plurality of datasets for creating the calibration model. The blood sugar value estimation apparatus is configured to set a reference spectrum by measuring a bio-spectrum of a person being tested. The blood sugar value estimation apparatus is configured to calculate a difference spectrum which is a difference between "the reference spectrum" and "a measurement spectrum which is measured in a time other than a time when the reference spectrum is measured". The blood sugar value estimation apparatus is configured to make a change of the calibration model, used in calculating the blood sugar value, according to a variation of the difference spectrum.

In addition to the above, it is preferred that the blood sugar value estimation apparatus is configured to make a plurality of change of the calibration models, within a period for measurement, according to the variation of the difference spectrum.

In addition, the blood sugar value estimation apparatus is preferably configured to vary the reference spectrum which is used for selection of the calibration model according to the variation of the difference spectrum after a certain period of time.

Preferably, when the blood sugar value estimation apparatus makes a plurality of the change of the calibration models within the period for measurement, the blood sugar value estimation apparatus calculates "a first estimated blood sugar value on the basis of the calibration model which is previously used" and calculates "a second estimated blood sugar value on the basis of the calibration model which is newly employed". The blood sugar value estimation apparatus is configured to make a bias correction such that the first estimated blood sugar value and the second blood sugar value are coincided with each other. Since then, the blood sugar value estimation apparatus estimates the blood sugar value.

It is preferred that the blood sugar value estimation apparatus is configured to make the change of the calibration model according to a development of a peak of a water in the difference spectrum. (The peak of the water corresponds to around 1450 nanometers.) Or, it is preferred that the blood sugar value estimation apparatus is configured to make the change of the calibration model according to a development of a peak of a fat in the difference spectrum. (The peak of the fat corresponds to around 1730 nanometers.)

In addition, the blood sugar value estimation apparatus may be make the variation of the calibration model according to a development of a peak of a water in the difference spectrum and a development of a peak of a fat in the difference spectrum.

A plurality of the calibration models or a plurality of the datasets for creating the calibration model may be created by a numerical simulation. Under this condition, the numerical simulation includes a disturbance having at least one of a variation of an optical constant of the water and a variation of an optical constant of the fat.

The calibration model corresponding to the development of the peak of the water or the datasets for creating the calibration model corresponding to the development of the peak of the water may be created by the numerical simulation. In this case, the numerical simulation is set to simulate a propagation of light through the skin tissue. In addition, the numerical simulation is set to include the disturbance which includes a variation of an optical constant which corresponds to a variation of an amount of water in the surface skin tissue. In addition, the numerical simulation may be set to simulate a propagation of the light through the skin tissue. Under the above condition, the numerical simulation is set to include the disturbance which is a variation of the optical constant corresponds to the variation of the concentration of the fat applied to the subcutaneous tissue. In addition, the numerical simulation may be set to simulate the propagation of the light in the skin tissue. Under the above condition, the numerical simulation is set to include the disturbance which is a variation of an optical constant which corresponds to a variation of the scattering characteristic applied to the surface skin tissue.

In addition, it is preferred that the blood sugar value estimation apparatus comprises a light source, a measurement probe, and a computing unit. The light source is configured to emit the light. The measurement probe is configured to receive the light. The measurement probe is configured to apply "the light which is received by the measurement probe" to the living body. When the light applied to the living body is diffusely reflected in the living body, a diffusely-reflected light is developed. The measurement probe is configured to receive the diffusely-reflected light. The computing unit is configured to extract "the optical spectrum of the living body" included in the diffusely-reflected light. The computing unit comprises the calibration model creating means.

In addition, the computing unit preferably comprises a measurement means and a blood sugar value estimation means. The measurement means is configured to extract "the optical spectrum of the living body" included in the diffusely-reflected light. The blood sugar value estimation means is configured to non-invasively calculate the blood sugar value with time on the basis of "the optical spectrum, of the living body, measured by the measurement means" with time and "the calibration model".

Preferably, the calibration model creating means comprises a reference spectrum setting means, a measurement spectrum setting means, a difference spectrum calculating means, and a calibration model changing means. The reference spectrum setting means is configured to measure the bio-spectrum of the person being tested to set the reference spectrum. The measurement spectrum setting means is configured to measure the bio-spectrum in a time different from a time when the reference spectrum is measured to set the measurement spectrum. The difference spectrum calculating means is configured to calculate the difference spectrum which is a difference between the measurement spectrum and the reference spectrum. The calibration model changing means is configured to change the calibration model for calculating according to the variation of the difference spectrum.

In addition, it is preferred that the measurement spectrum setting means is configured to set the measurement spectrum in present as a first measurement spectrum. The measurement spectrum setting means is configured to set the measurement spectrum of a previous time as a second measurement spectrum. The difference spectrum calculating means is configured to calculate a first difference spectrum which is defined by a difference between the first measurement spectrum and the reference spectrum. The difference spectrum calculating means is configured to calculate a second difference spectrum which is defined by a difference between the second measurement spectrum and the reference spectrum. The difference spectrum calculating means is configured to calculate a variation of a difference spectrum which is defined by a difference between the first difference spectrum and the second difference spectrum. The calibration model changing means is configured to make the change of the calibration model for calculating on the basis of the variation of the difference spectrum.

In addition, the following configuration is also preferably employed. That is, the measurement spectrum setting means is configured to set the measurement spectrum in present as a first measurement spectrum. The measurement spectrum setting means is configured to set the measurement spectrum which is measured in a previous time from the present time as a second measurement spectrum. The reference spectrum setting means is configured to measure "the bio-spectrum of the person being tested" with every a first predetermined period of time after "setting the reference spectrum again to set the reference spectrum which is measured by the reference spectrum setting means". The reference spectrum measurement means is configured to set a first reference spectrum which is defined by the reference spectrum which is measured before a second predetermined period of time from a time when the reference spectrum measurement means measures the first measurement spectrum. The reference spectrum measurement means is configured to set a second reference spectrum which is defined by the reference spectrum which is measured by the reference spectrum measurement means in a previous time from a time when the first reference spectrum is measured. The difference spectrum calculating means is configured to calculate the first difference spectrum which is a difference between the first measurement spectrum and the first reference spectrum. The difference spectrum calculating means is configured to calculate a second difference spectrum which is a difference between the second measurement spectrum and the second reference spectrum. The difference spectrum calculating means is configured to calculate a variation of the difference spectrum which is a difference between the first difference spectrum and the second difference spectrum. The calibration model changing means is configured to change the calibration model for calculating on the basis of the variation of the difference spectrum.

In addition, it is preferred that the first reference spectrum satisfies two conditions including (1) and (2). (1) The first reference spectrum corresponds to the reference spectrum which is measured at a time before a time when the first measurement spectrum is measured. (2) The first reference spectrum corresponds to a reference spectrum which is latest among " the reference spectrums measured at a time before a time when the first measurement spectrum is measured" .

It is preferred that the blood sugar value estimation means is configured to non-invasively calculate the blood sugar value with time on the basis of "a predetermined calibration model" and "the optical spectrum of the living body which is measured by the measurement means with time" when the difference spectrum calculating means calculates no difference spectrum.

It is preferred that the calibration model changing means is configured to create a predetermined calibration model from a plurality of the calibration models or a plurality of the datasets for creating the calibration model when the difference spectrum calculating means calculates no difference between the difference spectrums. According to this, the blood sugar value estimation means is configured to non-invasively calculate the blood sugar value with time on the basis of the predetermined calibration model and the optical spectrum of the living body which is measured by the measurement means with time when the difference spectrum calculating means calculates no difference spectrum.

### EFFECT OF THE INVENTION

In this invention, the calibration model used in the calculation for estimation of the blood sugar value is changed according to the variation of the difference spectrum. Therefore, it is possible to select the suitable calibration model at suitable time. This results in improvement of the estimation accuracy of the blood sugar value. Particularly, the blood sugar value estimation apparatus is preferably used to monitor the blood sugar value with time, exceedingly.

### BRIEF EXPLANATION OF THE DRAWING

Fig. 1 shows a flow chart indicating the operation in one example of the embodiment of this invention.
Fig. 2 shows a schematic drawing of the blood sugar value estimation apparatus of this invention.
Fig. 3 shows a schematic drawing of the model of the light emitting and the light receiving system in the simulation of this invention.
Fig. 4 shows an explanation drawing of the absorption constant of the skin tissue used in the simulation of this invention.
Fig. 5 shows an explanation drawing of the scatter coefficient of the skin tissue used in the simulation of this invention.
Fig. 6 A to Fig. 6 H show explanation drawings indicating the variation with time of the difference spectrum in the example.
Fig. 7 shows an explanation drawing comparing the estimated blood sugar value and the actually measured blood sugar value in the example.
Fig. 8 A to Fig. 8 H show explanation drawings indicating the variation with time of the difference spectrum in another example.
Fig. 9 shows an explanation drawing comparing the estimated blood sugar value with the actually measured blood sugar value in the example.
Fig. 10 shows an explanation drawing comparing the estimated blood sugar value and the actually measured blood sugar value in the comparative example.
Fig. 11 shows a block diagram of the arithmetic circuit.

### BEST MODE FOR CARRYING OUT THE INVENTION

The blood sugar value estimation apparatus in this embodiment is hereinafter explained. Fig. 2 shows a schematic drawing of the blood sugar value estimation apparatus. As will be understood from Fig. 2, the blood sugar value estimation apparatus in this embodiment comprises a halogen lamp 1, the heat shield plate 2, the lens 4, the light fiber bundle 5, the measurement probe 9, the reference probe 10, the measurement output member 11, the reference output member 12, the lens 13, the lens 22, the diffraction grating 14, the light receiving element 15, the A/D converter 16, and the computing unit 17.

The halogen lamp 1 is, so called, the light source. Therefore, although Fig. 2 discloses the halogen lamp 1, the light source is not limited to the halogen lamp 1. The light source is configured to emit the light. The light emitted from the light source 1 is applied to the heat shield plate 2. The lens 4 disposed on the opposite side of the heat shield plate 2 from the halogen lamp 1. The light emitted from the halogen lamp 1 passes through the pin hole 3 and then entered into the lens 4. The light entered into the lens is entered into the first end of the light fiber bundle 5.

The light fiber bundle 5 comprises the measurement light fiber 7 and the reference light fiber 8. The first end of the measurement light fiber 7 and the first end of the reference light fiber 8 are defined as the first end of the light fiber bundle 5. The measurement light fiber 7 is connected at its second end to the measurement probe 9. The measurement probe 9 is connected to the measurement output member 11 through the light fiber. The reference light fiber 8 is connected at its second end to the reference probe 10. The reference probe 10 is connected to the reference output member 12 through the light fiber.

The measurement output member 11 is optically connected to the A/D converter 16 through the lens 13, the diffraction grating 14, and the light receiving element 15. Furthermore, the reference output member 12 is also optically connected to the A/D converter 16 through the lens 13, the diffraction grating 14, and the light receiving element 15. The A/D converter is connected to the computing unit 17.

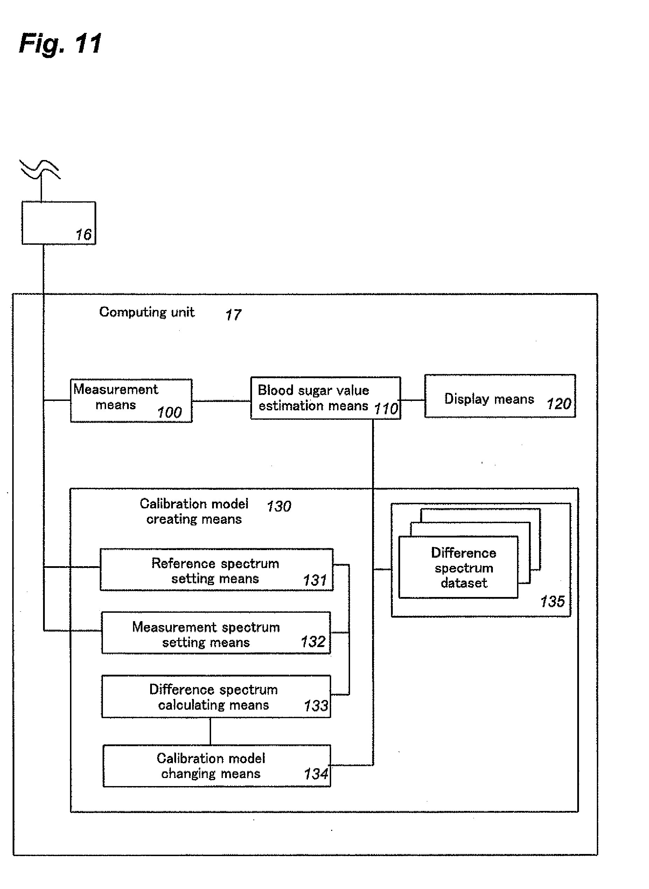

Fig. 11 shows a block diagram of the computing unit 17. As will be understood from Fig. 11, the computing unit 17 comprises a measurement means 100, the calibration model creating means 130, the blood sugar value estimation means 110, and the display means 120. The measurement means 100 is configured to receive the first signal. The measurement means 100 is configured to measure the optical spectrum of the living body from the first signal. In other words, the measurement means 100 is configured to extract the optical spectrum of the living body from the first signal. The optical spectrum of the living body is exemplified by the peak wavelength of the light absorbed by the water and the peak wavelength of the light absorbed by the fat. The peak wavelength of the light absorbed by the water is 1430 nanometers or more and 1480 nanometers or less. The peak wavelength of the light absorbed by the fat is 1670 nanometer or more and 1780 nanometer or less. In addition, it is possible to employ both the peak wavelength of the light absorbed by the water and the peak wavelength of the light absorbed by the fat as the optical spectrum. The calibration model creating means 130 comprises the reference spectrum setting means 131, the measurement spectrum setting means 132, the difference spectrum calculating means 133, the calibration model changing means 134, and the difference spectrum dataset 135.

The reference spectrum setting means 131 is configured to receive the first signal. The reference spectrum setting means 131 is configured to measure the optical spectrum of the living body from the first signal. In other words, the reference spectrum setting means 131 is configured to extract the optical spectrum of the living body from the first signal. The optical spectrum of the living body measured by the reference spectrum setting means 131 is set as the reference spectrum.

The measurement spectrum setting means 132 is configured to set "the optical spectrum of the living body measured from the first signal by the measurement spectrum setting means 132 after a predetermined period of time from when the computing unit 17 receives the first signal" as the measurement spectrum.

The difference spectrum calculating means 133 is configured to calculate the difference spectrum which is a difference between the reference spectrum and the measurement spectrum. Then, the difference spectrum calculating means 133 is configured to calculate the difference spectrum with time. The difference spectrum calculating means 133 is configured to calculate the difference of the difference spectrums to obtain the variation of the difference spectrum.

The calibration model changing means 134 is configured to change the calibration model for the calculation according to the variation of the difference spectrum.

The blood sugar value estimating means 110 is configured to estimate the blood sugar value from "the optical spectrum of the living body measured by the measurement means 100" and "the calibration model which is changed by the calibration model changing means 134".

The display means 120 is configured to display the blood sugar value estimated by the blood sugar value estimation means 110.

In this manner, the calibration model creating means 130 is configured to measure the living body spectrum of the person being tested to set the reference spectrum. The calibration model creating means 130 is configured to obtain the difference spectrum which is a difference between " the reference spectrum" and " the measurement spectrum which is measured in a time other than a time when the reference spectrum is measured" . The calibration model creating means 130 is configured to vary the calibration model used in the calculation according to the variation of the difference spectrum.

Then, the computing unit 17 estimates the blood sugar value from the optical spectrum of the living body and the calibration model. The computing unit 17 is configured to allow the display device to display "the blood sugar value which is estimated by the computing unit 17".

Such the blood sugar value estimation device is operated as follows. Firstly, when the light source emits the light, the light passes through the pin hole 3 and enters into the lens 4. The light entered into the lens 4 is entered into the light fiber bundle 5 from the first end of the light fiber bundle 5. The light entered into the light fiber bundle 5 passes through the measurement light fiber 7 and the reference light fiber 8. The light passing through the measurement fight fiber 7 is applied to the human body from the measurement probe 9. The light applied to the human body is dififusely-reflected in the human body. Consequently, the diffusely-reflected light is developed. The measurement probe 9 receives the diffusely-reflected light. The diffusely reflected light received by the measurement probe 9 is sent to the measurement output member 11 through the light fiber. When the light is emitted from the measurement output member 11, the light is applied to the light receiving element 15 through the lens 22 and the diffraction grating 14. When the light receiving element 15 receives the light, the light receiving element 15 generates the electrical signal corresponding to the diffusely reflected light. Then, the light receiving element 15 sends the electrical signal to the A/D converter 16. The A/D converter 16 is configured to make an analog-to-digital conversion of the electrical signal corresponding to the diffusely reflecting light to create the first signal. The first signal is sent from the A/D converter 16 to the measurement means 100, the reference spectrum setting means 131, and the measurement spectrum setting means 132 of the computing unit 17.

When the measurement means 100 receives the first signal, at first, the measurement means 100 extracts the optical spectrum of the living body from the first signal. The measurement means 100 send the optical spectrum of the living body to the blood sugar value estimation means 110. The blood sugar value estimation means 110 calculates the blood sugar value, non-invasively, on the basis of the optical spectrum and the calibration model. It is noted that, when the blood sugar value estimation means 110 receives the optical spectrum, the difference spectrum calculating means 133 does not yet calculate the difference spectrum. Therefore, the calibration model changing means 134 is not capable of varying the calibration model on the basis of the difference of the difference spectrums. Therefore, the blood sugar value estimation means 110 is configured to estimate the blood sugar value on the basis of the first optical spectrum and the initial calibration model created by the predetermined difference spectrum dataset 135. Then, the blood sugar value which is estimated by the blood sugar value estimation means 110 is displayed on the display means 120.

In addition, when the measurement means 100 extracts the optical spectrum from the first signal, the reference spectrum setting means 131 also extracts the optical spectrum from the first signal. Then, the reference spectrum setting means 131 sets the optical spectrum as the reference spectrum.

Then, the measurement means 100 extracts the optical spectrum of the living body from the first signal every five minutes. Then, the blood sugar value estimation means 110 estimates the blood sugar value from "the optical spectrum extracted every five minutes" and "the calibration model created on the basis of the difference spectrum dataset 135 which is predetermined". The blood sugar value estimation means 110 estimates the blood sugar value every five minutes. That is, the blood sugar value estimation means 110 non-invasively estimates the blood sugar value, with time from "the optical spectrum of the living body measured every five minutes and the initial calibration model created on the basis of the difference spectrum dataset 135 which is predetermined".

Then, when thirty minutes are passed after the reference spectrum setting means 131 sets the reference spectrum, the measurement spectrum setting means 132 sets the second measurement spectrum. The second measurement spectrum is the optical spectrum of the living body measured by the measurement spectrum setting means 132 when the thirty minutes are passed from the setting of the reference spectrum by the reference spectrum setting means 131

Then, when the sixty minutes are passed after setting the reference spectrum by the reference spectrum setting means 131, the measurement spectrum setting means 132 sets the first measurement spectrum which is defined by the optical spectrum of the living body measured by the measurement spectrum setting means 132 when sixty minutes are passed after setting the reference spectrum by the reference spectrum setting means 131.

Therefore, the second measurement spectrum corresponds to the measurement spectrum which is measured in last time before the first measurement spectrum.

The difference spectrum calculating means 133 is configured to calculate the difference between the second measurement spectrum and the reference spectrum. Consequently, the difference spectrum calculating means 133 calculates the second difference spectrum. In addition, the difference spectrum calculating means 133 is configured to calculate the difference between the first measurement spectrum and the reference spectrum. Consequently, the difference spectrum calculating means 133 calculates the first difference spectrum. Then, the difference spectrum calculating means 133 calculates the difference between the first difference spectrum and the second difference spectrum. The difference spectrum calculating means 133 is configured to calculate the variation of the difference spectrum which is defined by the difference between the first difference spectrum and the second difference spectrum.

The calibration model changing means 134 is configured to select "the difference spectrum dataset 135 which corresponds to the variation of the difference spectrum" from "a plurality of the difference spectrum dataset 135". Then, the calibration model changing means 134 creates the calibration model on the basis of the difference spectrum dataset 135 which is selected. The latest calibration model which is created by the calibration model changing means 134 is sent to the blood sugar value estimation means 110 from the calibration model changing means 134.

When the blood sugar value estimation means 110 receives the latest calibration model from the calibration model changing means, the blood sugar value estimation means 110 changes the initial calibration model into the latest calibration model. The blood sugar value estimation means 110 estimates the blood sugar value non-invasively, with time, on the basis of "the optical spectrum of the living body measured every five minutes" and "the latest calibration model". The blood sugar value which is estimated with time is displayed on the display means 120 with time.

In addition, the above mentioned operation is continuously performed. That is, the calibration model is changed into the calibration model which reflects the latest disturbance every thirty minutes. In addition, the optical spectrum of the living body is measured every five minutes. The blood sugar value estimation means 110 estimates the blood sugar value on the basis of "the latest calibration model" and "the optical spectrum of the living body measured every five minutes".

It is noted that the measurement spectrum setting means 132 is configured to repeat setting the measurement spectrum every thirty minutes after the reference spectrum setting means 131 sets the reference spectrum. However, the intervals for repeat setting the measurement spectrum are not limited to thirty minutes. It is possible to set the intervals arbitrarily, and to set the intervals, for example, from one minute to one hundred minutes.

In addition, the measurement means 100 measures the optical spectrum of the living body every five minutes. However, the intervals for measurement of the optical spectrum of the measurement means are not limited to five minutes. For example, it is possible to set the interval arbitrarily, and to set the interval, for example, from one minute to one hundred minutes.

In addition, when the light is emitted from the light source, the light passes through the pin hole 3 and enters into the lens 4. The light which enters into the lens 4 enters into the light fiber bundle 5 from the first end of the light fiber bundle 5. The light which enters into the light fiber bundle 5 passes through the measurement light fiber 7 and the reference light fiber 8. The light which passes the reference light fiber 8 is applied to the reference plate 18 through the reference probe 10. The light which is applied to the reference plate 18 is reflected by the reference plate 18, whereby the reflected light is developed. The reference probe 10 receives the reflected light. The reflected light which is received by the reference probe 10 is sent to the measurement output member 12 through the light fiber. The light output from the measurement output member 12 is applied to the light receiving element 15 through the lens 13 and the diffraction grating 14. When the light receiving element 15 receives the light, the light receiving element 15 generates the electrical signal corresponding to the reflected light. Then, the light receiving element sends the electrical signal to the A/D converter 16. The A/D converter 16 makes an analog to digital conversion of the electrical signal corresponding to the reflected light into the second signal. The computing unit 17 exactly and non-invasively calculates the blood sugar value, with time, on the basis of the optical spectrum of the living body included in the first signal, the calibration model, and the optical spectrum which corresponds to the optical spectrum of the living body in the first signal and which is included in the second signal.

The embodiment of this invention is explained with one example. This invention is one which is configured to estimate the blood sugar value by measuring the near infrared ray spectrum with respect to the skin tissue. The skin tissue of the living body is mainly categorized as three tissues. The three tissues include a surface skin, an inner skin, and a subcutaneous tissue. The surface skin is a tissue which includes a stratum corneum. The blood capillary in the surface skin is not well developed. In addition, the inner skin mainly includes the fat tissue. Therefore, it is expected that the correlation between the hydrosoluble concentration of constituents of the living body in the above two tissue and the blood glucose level (blood sugar value) is low. Especially, it is expected that the correlation between the glucose concentration and the blood glucose level (blood sugar value) is low.

In contrast, the blood capillary of the inner skin tissue is developed. In addition, the concentration of constituents of the living body having the high hydrosoluble property has a high permeability with respect to the inner skin tissue. Especially, the glucose has a high permeability with respect to the inner skin tissue. Therefore, the concentration of constituents of the living body in the tissue is varied according to the blood sugar value, similar to the ISF. (ISF means Interstitial Fluid.) Especially, the concentration of the glucose is varied according to the blood sugar value. Therefore, in this invention, in order to make a spectrum measurement with respect to the inner skin tissue, the apparatus similar to the apparatus shown in Fig. 2 is used. Especially the near infrared ray having the wavelength of 1300 nanometers or more and 2500 nanometers or less is used. In addition, when measuring the near infrared spectrum, "the measurement probe for measuring the near infrared spectrum" used to be contacted to the skin. "The measurement probe for measuring the near infrared spectrum" having the light emitting member and the light receiving member is employed. The center-to-center distance of the light emitting member and the light receiving member is set to be 0.65 millimeters.

In addition, in this invention, a plurality of the calibration models (or a plurality of the datasets for creating the calibration model) are required. When creating the calibration model, it is effective to use the numerical simulation.

The difference of the form of the near infrared spectrum is caused by characteristics of the behavior of the skin such as an amount of water in the corneum, the fineness of the surface skin tissue, and the thickness of the skin tissue. However, when the spectrum is synthesized with using the numerical simulation, the characteristics of the behavior of the skin is represented by "the optical characteristic values such as an optical absorption coefficient, a scatter coefficient, and an anisotropic scatter coefficient parameter" of "the skin tissue including the surface skin, the inner skin, and the subcutaneous tissue".

The method of calculating the simulation spectrum is exemplified by the simulation using the stochastic method such as the Monte Carlo method and the random walk method and by the method of calculating the near infrared spectrum from the optical diffusion equation.

To explain the Monte Carlo method, the propagation of the near infrared ray through the medium (specifically, living tissue) is capable of being simulated by the mathematical scheme on the basis of the probability distribution of the absorption and the scattering of the near infrared ray. Therefore, in the actual calculation, the light is assumed to be a plurality of the light fluxes. Under this assumption, the propagation path of the light fluxes is determined on the basis of the optical property of the medium. Consequently, it is possible to recreate the near infrared spectrum under a predetermined light receiving and emitting condition.

As to the step of performing the simulation of the near infrared spectrum of the skin tissue, the steps of determining "the optical property such as the structure of the skin tissue, the absorption coefficient, the scatter coefficient, the refractive index, and the anisotropic scatter parameter of the measurement target" and "the photon number for calculation, and of performing the computing calculation", and calculating by the computer are performed.

The skin tissue comprises the surface skin tissue, the inner skin tissue, and the subcutaneous tissue layer. Therefore, when the simulation is made by the skin tissue, the layer structure defined by the inside portion from the subcutaneous tissue layer is made modelization simply. In addition, by determining the thickness of each layer, the absorption coefficient, the scattering coefficient, and the anisotropic scatter parameter, it is possible to recreate the near infrared spectrum of the skin tissue of the person being tested with using the numerical simulation. The photon number is ordinary determined by the number of the several hundred-thousands or several millions.

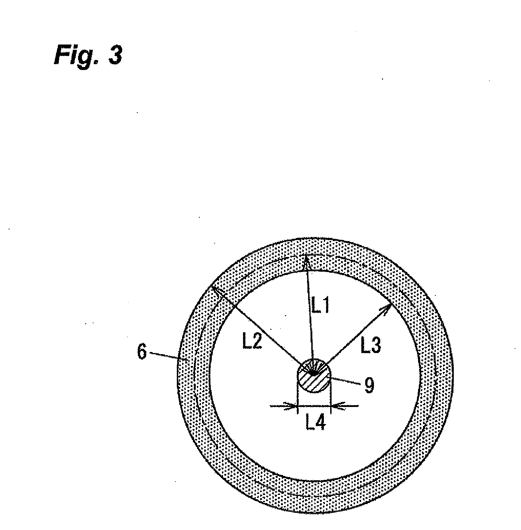

The following is the result of the example of the simulation according to the Monte Carlo method. This simulation is made with using the light emitting/light receiving model shown in Fig. 3. The light fiber for light emitting has a ring shape to have an external radius L2 of 0.7375 millimeters and an internal radius L3 of 0.5625 millimeters. The light fiber for light receiving has a circular shape to have an external diameter L4 of 0.175 millimeters. A light receiving/light emitting interval L is set to be 0.65 millimeters. Consequently, the structure of the probe shown in Fig. 2 is simulated.

According to the numerical simulation, the light fiber used in the example has NA (NA means numerical aperture) of 0.2. Therefore, the photon having the angle of 11.5 degrees, finally, is detected among the photon reached to the light fiber for detection. The input photon number is set to be one million. The skin structure used in the Monte Carlo method is set to have the surface skin tissue of 0.1 millimeters, the inner skin tissue of 0.9 millimeters, and the subcutaneous tissue layer of 2.0 millimeters. In addition, the layer which is lower than the subcutaneous tissue is defined as the perfect absorber.

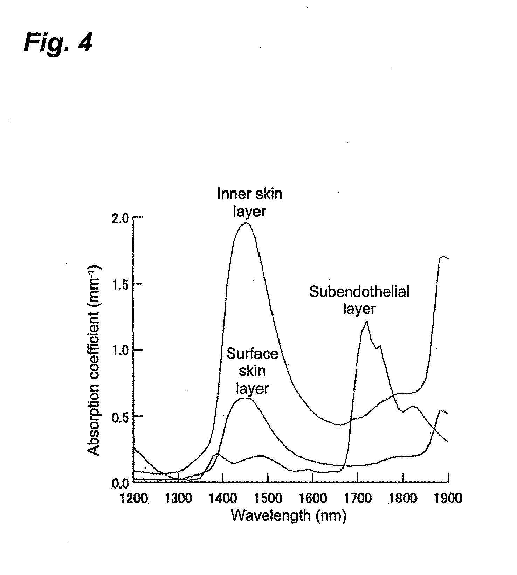

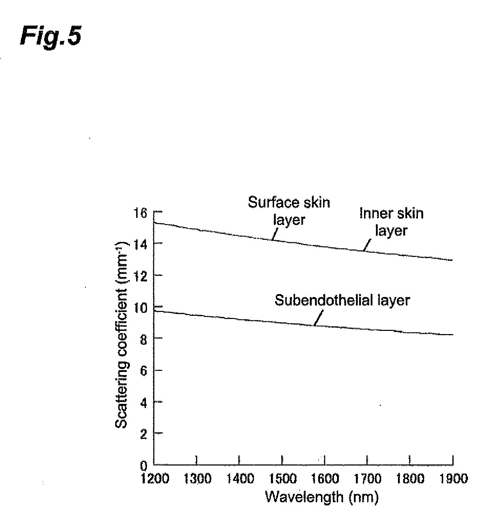

Fig. 4 and Fig. 5 show the optical characteristics of the skin tissues used in the simulation. In Fig. 4, the absorption coefficient of the inner skin tissue is made by overlapping the 60 percents of the water and the 15 percents of the protein. In addition, the absorption coefficient of the surface skin tissue is set to be 20 percents of the water. The absorption coefficient of the subcutaneous tissue layer is set to correspond to the absorption coefficient of the cholesterol. The scatter coefficients of the inner skin tissue and the subcutaneous tissue layer in Fig. 5 are set to be the scatter coefficient of the surface skin layer and the inner skin layer, respectively, which are determined by referring the literatures (non-patent literatures 1 and 2) of Troy and Simpson. The anisotropic scatter parameter of each the tissue is set to be 0.9. The refractive index of each the tissue is set to be 1.37. These are set to be constant with respect to the wave length.

Hereinafter, the invention is explained with examples.

### EXAMPLE 1

In the quantitative determination, the near infrared ray having the wavelength range of 1430 nanometers or more and 1850 nanometers or less was used. In the example, the measurement probe was worn to the left front arm of the person being tested. In addition, the example was made under a condition where the person being tested was in the sitting position with resting state. Under this condition, the blood sugar value was varied by performing the sugar tolerance test two times. The sugar tolerance test was performed by using the nutrition supplement of liquid type. (CALORIE MATE (name of commodity) by OTSUKA FOODS COMPANY, LIMITED) The actually measured blood sugar value was measured from the blood sampled from the puncture of the fingertip by using the simplified blood sugar measurement unit (DIASENSOR by ARKRAY).

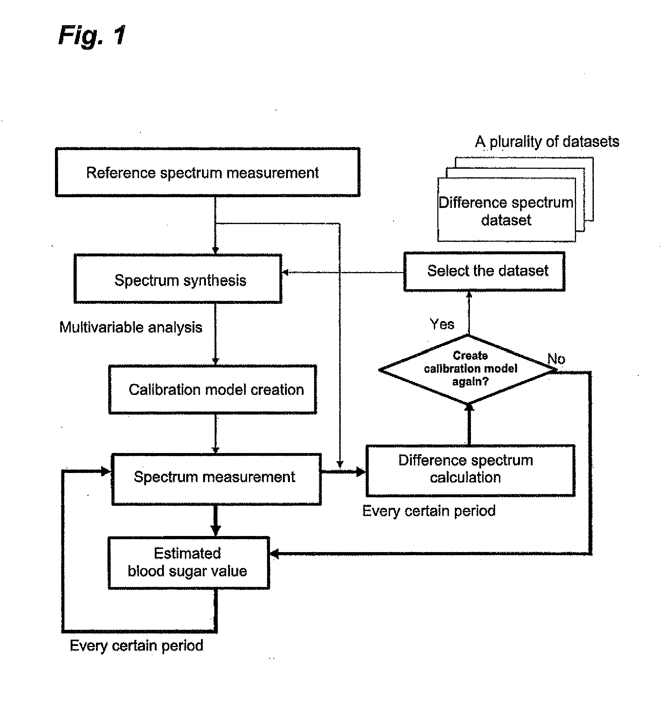

In this example, the actually measured spectrum was set to be reference spectrum as shown in the flow chart shown in Fig. 1. The calibration model used in the start of the example was obtained by multivariable analysis of the spectrum dataset created by adding the difference spectrum dataset with the reference spectrum. The difference spectrum dataset was prepared according to the numerical simulation, in advance, and was synthesized with the variation of the blood sugar value and the variation of the disturbance. A plurality of the difference spectrum datasets were prepared according to the kind of the disturbance to be combined.

When starting the example, there was no difference spectrum for considering the existence of the condition of the disturbance. Therefore, when starting the example, the difference spectrum dataset with the disturbance which was created under a condition where the variation of amount of the water was applied to the surface skin was selected from the difference spectrum datasets prepared in advance. The difference spectrum dataset was combined with element of the disturbance. The disturbance was the variation of the blood sugar value, an amount of the water, the concentration of the protein, and the concentration of the fat. In addition, the absorbance index and the scatter coefficient were varied on the basis of the assumption that the variation of the absorbance due to the variation of the above concentrations and the variation of the volume fraction due to the variation of the concentration of the protein and the fat were substituted to the water. In addition, the temperature variation was applied on the basis of the consideration of the peak shift of the water. In addition, the scatter coefficient and the anisotropic scatter parameter were varied independently from each other. It is noted that the method of applying the disturbance is not limited to the above.

In addition, the skin tissue being simulated was made modelization simply such that the skin tissue had a structure having the surface skin tissue (0.1 millimeters), the inner skin tissue (0.9 millimeters), the subcutaneous tissue layer (2.0 millimeters), and the perfect absorber below the subcutaneous tissue layer. The thickness of the skin was determined as the constant number. However, needless to say, it is possible to employ the thickness of the skin as the parameter, and calculate the regression model according to the operation similar to the above.

The calibration model was created on the basis of the spectrum dataset created by adding the difference spectrum dataset with the reference spectrum, and on the basis of the multivariable analysis which used the blood sugar value as the variable quantities of the target and used the simulation spectrum as the variable quantities of the explanation. As to the multivariable analysis, it is possible to use the multiple linear regression analysis, PLS regression analysis, main component regression analysis, and the neural net. In this example, the PLS regression analysis is used as the multivariable analysis.

The estimation of the blood sugar value by the near infrared ray was made by substituting the absorbance of each the wavelength of the actually measured spectrum every five minutes into the calibration model which was obtained.

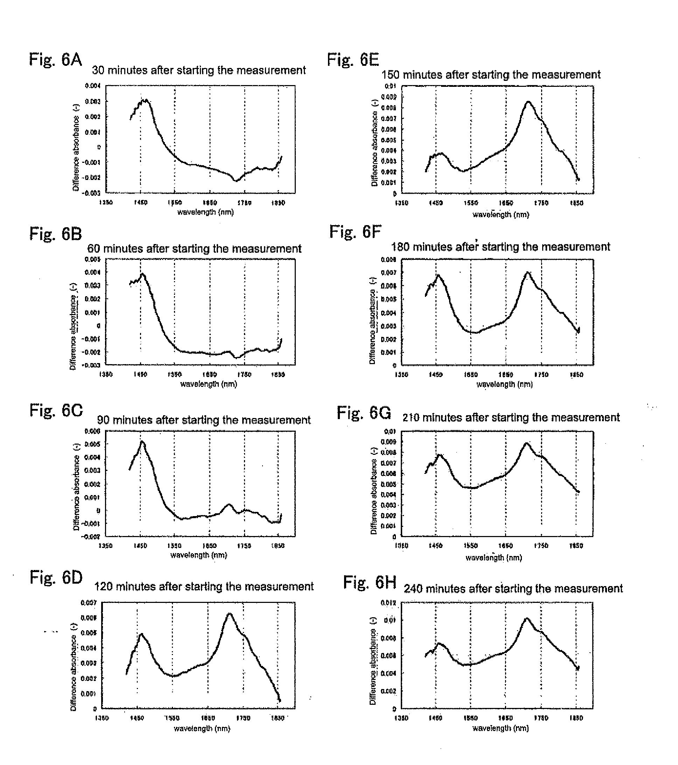

The consideration of the calibration model to be made by considering the spectrum variation obtained from the difference spectrum between the reference spectrum and the actually measured spectrum which was measured every thirty minutes. The variation with time of the difference spectrum in this example is shown in Fig. 6. Although the calibration model being used was judged every thirty minutes, the intervals are not limited thereto. It is possible to judge the calibration model when the judgment is required. In addition, it is possible to judge the calibration model every certain period of time.

In addition, in this example, the judgment of selecting the calibration model was performed on the basis of the peak wavelength (1730 nanometers) of the absorption by the fat. The judgment was simply performed on the basis of the fact whether the difference absorption in the wavelength of 1730 nanometers exceeds 0.002 or not. As will be understood from Fig. 6, until the judgment in three times (refer to Fig. 6 C), the peak development of the fat does not satisfy the judgment reference. Therefore, the blood sugar values were estimated by using the calibration model which was used in the start of the example, with no change. That is, the blood sugar value was estimated by using the calibration model created from the difference spectrum dataset with the disturbance which was defined by the fact that the variation of an amount of the water applied to the surface skin layer.

After four times of the judgment (refer to Fig. 6 D), the peak development of the fat exceeded the reference. Therefore, among the difference datasets prepared in advance, the difference dataset with the disturbance defined by the variation of the anisotropic scatter parameter (G factor) in the surface skin layer was used after four times of the judgment. "The element of the disturbance other than the anisotropic scatter parameter" incorporated into the difference spectrum dataset was similar to the element of the disturbance applied to the difference spectrum dataset with the disturbance when the variation of an amount of the water was applied to the surface skin layer. When the anisotropic scatter parameter (G factor) in the surface skin layer was varied as the disturbance, the light reaching condition of reaching the light into the skin tissue was varied. As a result, the effect with respect to the fat in the subcutaneous tissue was varied.

The calibration model was created by the multivariable analysis using the blood sugar value as the variable quantities of the target and using the simulation spectrum as the variable quantities of the explanation on the basis of the spectrum dataset created by adding the difference spectrum dataset with the reference spectrum, similar to the starting of the example.

In this example, two sets of the difference spectrum datasets were prepared in advance. The calibration model which was used in the estimation of the blood sugar value was judged according to the difference absorption of the absorption peak wavelength of the fat (1730 nanometers).

When the fourth judgment was performed, the blood sugar value was estimated by using the calibration model which was previously used and also the blood sugar value was estimated by using the calibration model which was newly created. Then, the bias correction was made such that the obtained two estimated blood sugar values coincide with each other. Subsequently, the blood sugar value was estimated by using the calibration model which was newly created.

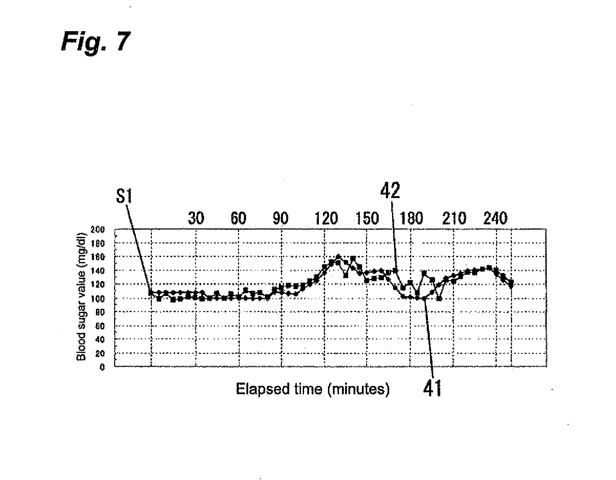

Fig. 7 shows the comparison of the estimated blood sugar value 42 which was measured by the above method and the actually measured blood sugar value 41 by the blood sampling. The reference character S1 in the illustration indicates the time of starting the reference spectrum. The coefficient of the correlation between the actually measured blood sugar value 41 and the estimated blood sugar value 42 was 0.81. The rate that the estimated blood sugar value 42 exists within the error of plus or minus 20 percents from the actually measured blood sugar value 41 was 92.3 percents.

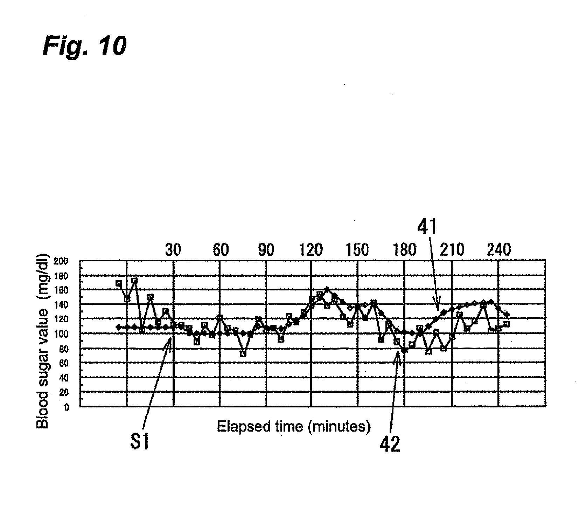

As to comparative example, the blood sugar value was estimated by using the single calibration model with using the experimental data same as the first example, without judging the spectrum variation. The calibration model being used was created according to the difference spectrum dataset with variation, similar to the example 1, of "the blood sugar value, an amount of the water, the concentration of the protein, the concentration variation of the fat, the temperature variation, the scattering coefficient, and the anisotropic scatter parameter" of the inner skin layer without applying "the disturbance with respect to the spectrum surface skin tissue". The step of creating the calibration model was same as the example. Fig. 10 shows the comparison of the estimated blood sugar value 42 which was estimated under the above condition and the actually measured blood sugar value 41 by the blood sampling. In the comparative example, the blood sugar value could not be estimated from the starting of the experiment such as the example 1. Therefore, the estimation of the blood sugar value was made with using the reference spectrum which is defined by the actually measured spectrum after thirty five minutes from the starting of the experiment. The correlation coefficient between the actually measured blood sugar value 41 and the estimated blood sugar value 42 in the comparative example was 0.36. The rate that the estimated blood sugar value 42 exists within the error of plus or minus 20 percents from the actually measured blood sugar value 41 was 70.6 percents.

### EXAMPLE 2

The step of estimating the blood sugar value in this example was same as the example 1. However, in this example, the difference absorption of the absorption peak wavelength of fat (1730 nanometers) was not use directly. The reference wavelength was set to be 1650 nanometers. (The reference wavelength was set as the minimum wavelength which had a smallest absorbance within the range within the wavelength of 1430 nanometers or more and 1850 nanometers or less.) The judgment for varying the calibration model which was used in the estimation of the blood sugar value was made by using the difference between the difference absorbance of the reference wavelength and the difference absorbance of the absorption peak wavelength. With regard to the variation with time of the absorption spectrum, there is a case that it is impossible to neglect the variation of the base line. In this case, to employ the difference between the reference wavelength in this example and the difference absorption of the absorption peak wavelength as the judgment reference results in the exact judgment. In terms of results, the estimation of the blood sugar value resulted in the estimation of the blood sugar value in the example 1. The setting of the reference wavelength was not limited to 1650 nanometers. It is possible to select the wavelength suitable for detection of the characteristics of the spectrum variation.

### EXAMPLE 3

The step of estimating the blood sugar value in this example was same as the example 1. The difference point was the fact that two wavelengths of absorption peak wavelength of the water (1450 nanometers) and the absorption peak wavelength of the fat (1730 nanometers) were used for selection of the calibration model. The necessity of varying the calibration model was judged according to the above two wavelengths. In the judgment, the magnitude of difference absorption of the absorption peak wavelength of the water and the magnitude of the difference absorption of the absorption peak wavelength of the fat were compared with each other. If the absorption peak wavelength of the water was greater, the calibration model which was created from the difference spectrum dataset with disturbance of the variation of an amount of the water in the surface skin layer was selected and simply used. If the absorption peak wavelength of the fat was greater, the calibration model which was created from the difference spectrum dataset with disturbance of the variation of anisotropic scatter parameter (G factor) in the surface skin was selected and simply used. The estimation of the blood sugar value results in the estimation of the blood sugar value same as the example 1.

### EXAMPLE 4

In this example, the step of estimating the blood sugar value was same as the example 1. However, the difference dataset which includes the disturbance of the variation of the anisotropic scatter parameter (G factor) in the surface skin layer was not used. The difference dataset with the variation of the fat concentration in the subcutaneous tissue was used. The judgment of the selection of the calibration was made similar to the example 1. Therefore, when the difference absorption of the absorption peak wavelength of the fat exceeded 0.002, the difference dataset in this example is used. The estimation of the blood sugar value resulted in the estimation of the blood sugar value equal to that of the example 1.

### EXAMPLE 5

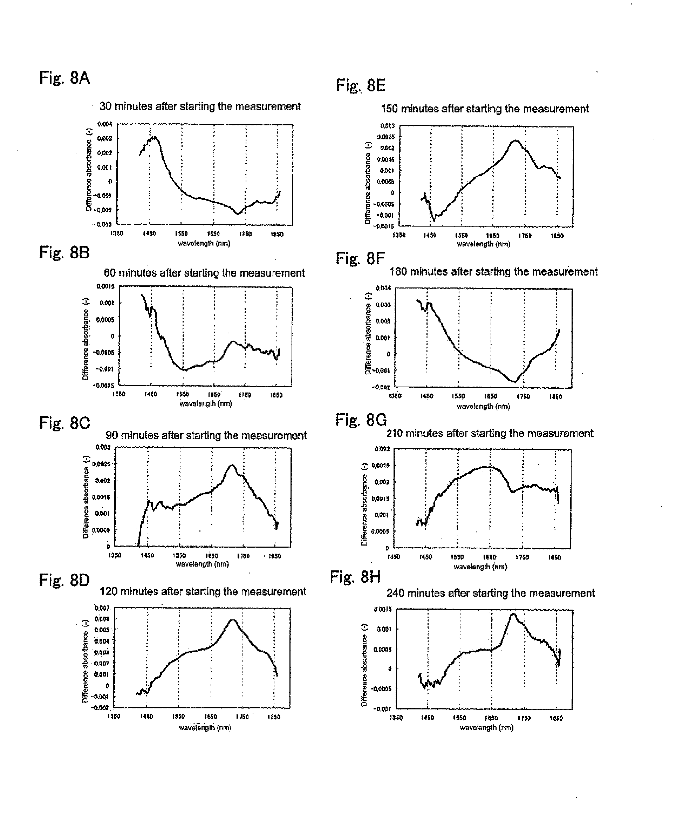

The step of estimation of the blood sugar value in this example was similar to the step in the example 1. The difference point was the fact that the reference spectrum was varied every thirty minutes. In addition, the judgment of the variation of the calibration model was made according to the form of the difference spectrum between the reference spectrum and the actually measured spectrum which was measured after thirty minutes of the measurement of the reference spectrum. The variation with time of the difference spectrum in this example was indicated in Fig. 8. The update of the reference spectrum was made every thirty minutes. However, the time interval is not limited to the above. If the reference spectrum is set according to the necessary, there is no need to update the reference spectrum at a certain interval.

The difference dataset prepared in this example comprises two difference datasets. One of the two difference datasets was the difference spectrum dataset with the disturbance of the variation of an amount of the water to the surface skin layer, and the other of the two difference datasets was the difference spectrum dataset with the disturbance of the anisotropic scatter parameter (G factor) in the surface skin layer.

Similar to the example 1, when starting the experiment, there is no difference spectrum for judging the disturbance condition. Therefore, the difference spectrum dataset with the disturbance of the variation of an amount of the water applied to the skin surface layer was used from a plurality of the difference spectrum datasets.

In addition, in this example, the judgment of the selection of the calibration model was made on the basis of the absorption wavelength (1450 nanometers) of the water. This judgment was a simple judgment of judging whether the difference absorption between the difference absorption of the absorption peak wavelength (1450 nanometers) of the water and the reference wavelength (1650 nanometers) was positive or negative.

As will be understood from Fig. 8, until the second judgment, the value that the reference wavelength was subtracted from the absorption peak wavelength of the water was positive. Similarly, in the sixth judgment, the value that the reference wavelength was subtracted from the absorption peak wavelength of the water was positive. Therefore, the estimation of the blood sugar value was made by using the calibration model created from the difference spectrum dataset with the disturbance of the variation of an amount of the water applied to the surface skin layer. In the third judgment, in the fifth judgment, and after the seventh judgment, the value that the reference wavelength was subtracted from the difference absorption of the absorption peak wavelength of the water was negative. Therefore, the estimation of the blood sugar value was made by using the difference dataset which was created by the difference dataset which was prepared in advance with the disturbance of the variation of the anisotropic scatter parameter (G factor) applied to the surface skin layer.

The reference spectrum which was added to the difference spectrum dataset is varied every thirty minutes. Consequently, the calibration model which was created with using the difference spectrum according to the above judgment reference is created every thirty minutes.

In this example, when the calibration model was created, the blood sugar value was estimated by using the calibration model which was previously used. In addition, the blood sugar value was estimated by using the calibration model of newly created. In addition, the bias correction is made such that the two estimated blood sugar values were coincided with each other. Consequently, the newly created calibration model was used to estimate the blood sugar value.

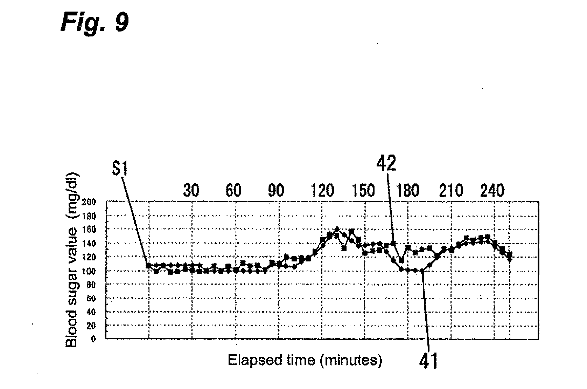

Fig. 9 shows an estimated blood sugar value 42 created by the above method and an actually measured blood sugar value 41 obtained by the blood sampling. The correlation coefficient between the actually measured blood sugar value 41 and the estimated blood sugar value in this example was 0.81. In addition, the rate of the estimated blood sugar value which exists within the error of plus or minus 20 percents from the actually measured blood sugar value was 88.5 percents.

This invention has the objective of estimating the blood sugar value in the above mentioned. However, the technology indicated in the above is not limited thereto. That is it is possible to apply the above technology to the estimation of the physiological index such as uric acid level, an amount of cholesterol, an amount of the neutral fat, an amount of the albumin content, an amount of globulin content, oxygen saturation, an amount of hemoglobin, and myoglobin content.

As explained above, the blood sugar value estimation apparatus in this invention is configured to non-invasively calculate the blood sugar value with time on the basis of the optical spectrum, of the living body, measured with time and the calibration model. The blood sugar value estimation apparatus comprises the calibration model creating means. The calibration model creating means is configured to create the calibration model from a plurality of the calibration models or a plurality of datasets for creating the calibration model. The calibration model creating means is configured to measure the bio-spectrum of a person being tested to set a reference spectrum. The calibration model creating means is configured to calculate the difference spectrum which is defined by the difference between "the reference spectrum" and "the measurement spectrum which is measured in a time other than a time when the reference spectrum is measured". The calibration model creating means is configured to change the calibration model for calculating according to a variation of the difference spectrum.

Consequently, the blood sugar value estimation apparatus may perform a high accurate estimation of the blood sugar value which is a minor component. Specifically, it is possible to perform a high accurate estimation of the blood sugar value when monitoring the blood sugar value which is a minor component with time.

Furthermore, the blood sugar value estimation apparatus is configured to make a plurality of the changes of the calibration model according to the variation of the difference spectrum. Consequently, it is possible to arbitrarily select the calibration model corresponding to the disturbance which is varied. This results in the accurate estimation of the blood sugar value.

In addition, the blood sugar value estimation apparatus is configured to change the reference spectrum which is used when selecting the calibration model corresponding to the variation of the difference spectrum after passing a predetermined period of time from the measurement of the reference spectrum. Consequently, the reference spectrum is updated as needed. Therefore, the calibration model is created on the basis of the reference spectrum which is updated as needed. This results in the accurate estimation of the blood sugar value.

In addition, the blood sugar value estimation apparatus is configured to calculate the estimated values on the basis of the calibration model which is previously used when estimating the value, and is configured to calculate the estimated value on the basis of the calibration model which is newly used. Then, the blood sugar value estimation apparatus is configured to make a bias correction such that the " two estimated values which are estimated" are coincided with each other. After the bias correction of the blood sugar estimation apparatus, the blood sugar value estimation apparatus estimates the blood sugar values thereafter. Consequently, it is possible to estimate the blood sugar value accurately.

In addition, the blood sugar value estimation apparatus further comprises a light source, a measurement probe, and a computing unit. The light source is configured to emit the light. The measurement probe is configured to receive the light. The measurement probe is configured to apply the light which is received by the measurement probe, whereby the light applied to the living body is diffusely reflected by the living body. Consequently, the diffusely reflected light is developed. The measurement probe is configured to receive the diffusely reflected light. The computing unit is configured to extract "the optical spectrum of the living body" included in the diffusely reflected light. The computing unit comprises the calibration model creating means.

In addition, the computing unit comprises the measurement means and the blood sugar value estimation means. The measurement means is configured to extract "the optical spectrum of the living body" included in the diffusely reflected light. The blood sugar value estimation means is configured to non-invasively calculate the blood sugar value with time on the basis of "the optical spectrum of the living body which is measured with time by the measurement means" and "the calibration model".

In addition, the calibration model creating means comprises the reference spectrum setting means, the measurement spectrum setting means, a difference spectrum calculating means, and the calibration model changing means. The reference spectrum setting means is configured to measure the bio-spectrum of the person being tested to set the reference spectrum. The measurement spectrum setting means is configured to set the measurement spectrum which is defined by the bio-spectrum which is measured at a time other than a time when the reference spectrum is measured. The difference spectrum calculating means is configured to calculate the difference spectrum which is a difference between the measurement spectrum and the reference spectrum. The calibration model changing means is configured to change the calibration model for calculating according to the variation of the difference spectrum.

The measurement spectrum setting means is configured to set the first measurement spectrum which is defined by the measurement spectrum at present. In addition, the measurement spectrum setting means is configured to set the second measurement spectrum which is defined by the measurement spectrum of previous time of the measurement spectrum at present. The difference spectrum calculating means is configured to calculate the first difference spectrum which is a difference between the first measurement spectrum and the reference spectrum. The difference spectrum calculating means is configured to calculate the second difference spectrum which is a difference between the second measurement spectrum and the reference spectrum. The difference spectrum calculating means is configured to calculate the variation of the difference spectrum which is a difference between the first difference spectrum and the second difference spectrum. The calibration model changing means is configured to change the calibration model for calculating according to the variation of the difference spectrum. Consequently, it is possible to select, as needed, the calibration model corresponding to the disturbance which is varied. Therefore, this results in the accurate estimation of the blood sugar value.

In addition, in the example 5, the reference spectrum is varied every thirty minutes. The judgment of the variation of the calibration model is made according to the form of the difference spectrum of "the actually measured spectrum which is measured after thirty minutes from the measurement of the reference spectrum" with respect to "the reference spectrum". That is, "the reference spectrum which is measured after the thirty minutes of the measurement of the actually measured spectrum" is used as the first reference spectrum. "The reference spectrum which is measured after thirty minutes of the measurement of the first reference spectrum" is used as the second reference spectrum.

That is, as will be understood from the example 5, the measurement spectrum setting means is configured to set "the measurement spectrum at present" as "the first measurement spectrum". The measurement spectrum setting means is configured to set "the measurement spectrum of the previous time of the measurement spectrum at present" as "the second measurement spectrum". The reference spectrum setting means is configured to repeat measuring the bio-spectrum of the persons being tested with every a first predetermined period of time after setting the reference spectrum to set the reference spectrum. The reference spectrum measurement means is configured to set the first reference spectrum which is defined by "the reference spectrum which is measured before a second predetermined period of time before the measurement spectrum setting means measures the first measurement spectrum". The reference spectrum measurement means is configured to set the second reference spectrum which is defined by " the reference spectrum which is set at a previous time of the first reference spectrum" . The difference spectrum calculating means is configured to calculate the first difference spectrum which is a difference between the first measurement spectrum and the first reference spectrum. The difference spectrum calculating means is configured to calculate the second difference spectrum which is a difference between the second measurement spectrum and the second reference spectrum. The difference spectrum calculating means is configured to calculate the variation of the difference spectrum which is a difference between the first difference spectrum and the second difference spectrum. The calibration model changing means is configured to vary the calibration model for calculating according to the variation of the difference spectrum. Consequently, the reference spectrum and the measurement spectrum are updated, as needed. Therefore, the difference spectrum is also updated, as needed. As a result, the calibration model is created on the basis of the variation of the difference spectrum which is updated. In addition, the blood sugar value estimation apparatus non-invasively calculate the blood sugar value with time on the basis of the calibration model which is updated and the optical spectrum of the living body. Therefore, it is possible to estimate the accurate blood sugar value.

In addition, it is preferred that the first reference spectrum is the reference spectrum which is measured in a previous time before the first measurement spectrum is measured. In addition, the first reference spectrum is the latest reference spectrum among the reference spectrums which are measured in a previous time before the first measurement spectrum is measured.

In addition, the blood sugar value estimation means is configured to non-invasively calculate the blood sugar value with time on the basis of the calibration model which is predetermined and the optical spectrum of the living body measured with time by the measurement means when the difference spectrum calculating means calculates no difference spectrum. In this case, the blood sugar value estimation apparatus is capable of estimating the blood sugar value even if the difference spectrum calculating means does not calculate the difference of the difference spectrums.

The calibration model changing means is configured to create a predetermined calibration model from a plurality of the calibration models or a plurality of the datasets for creating the calibration model when the difference spectrum calculating means calculates no difference between the difference spectrums. The blood sugar value estimation means is configured to non-invasively calculate the blood sugar value with time on the basis of the calibration model and the optical spectrum of the living body which is measured by the measurement means with time when the difference spectrum calculating means does not calculate the difference spectrum. In this case, the blood sugar value estimation apparatus is capable of estimating the blood sugar value under a condition where the difference spectrum calculating means does not calculate the difference spectrum.

In addition, as will be understood from the example 1, the variation of the calibration mode for calculating is performed according to the development of the peak of the water in the difference spectrum. In other words, the changing of the calibration model for calculating is performed according to the variation of the peak wavelength of the light which is absorbed by the water. In the example 1, the peak wavelength of the light which is absorbed by the water is set as 1450 nanometers. However, the peak wavelength of the light absorbed by the water is not limited to 1450 nanometers. Specifically, the peak wavelength of the light absorbed by the water may have a range of 1430 nanometers or more and 1480 nanometers or less.

In addition, as shown in the example 2, the changing of the calibration model for calculating is performed according to the development of the peak of the fat in the difference spectrum. In other words, the changing of the calibration model for calibration is performed according to the variation of the peak wave length of the light absorbed by the fat in the difference spectrum. In the example 2, the peak wavelength of the light absorbed by the fat is set as 1730 nanometers. However, the peak wavelength of the light absorbed by the fat is not limited to 1730 nanometers. Specifically, the peak wavelength of the light absorbed by the fat may have a range of 1670 nanometers to 1780 nanometers.

## Claims

1. A blood sugar value estimation apparatus for non-invasively calculate a blood sugar value with time on the basis of an optical spectrum, with time, of a living body, and a calibration model,
said blood sugar value estimation apparatus comprising:
a calibration model creating means configured to create the calibration model from a plurality of the calibration models or a plurality of datasets for creating the calibration model,
wherein
said blood sugar value estimation apparatus is configured to set a reference spectrum by measuring a bio-spectrum of a person being tested,
said blood sugar value estimation apparatus is configured to calculate a difference spectrum which is a difference between the reference spectrum and a measurement spectrum which is measured in a time other than a time when the reference spectrum is measured,
said blood sugar value estimation apparatus is configured to make a change of the calibration model, used in calculating, according to a variation of the difference spectrum.

2. The blood sugar value estimation apparatus as set forth in claim 1, wherein
said blood sugar value estimation apparatus is configured to make a plurality of the changes of the calibration models, within a period for measurement, according to the variation of the difference spectrum.

3. The blood sugar value estimation apparatus as set forth in claim 1 or 2, wherein
the blood sugar value estimation apparatus is configured to vary the reference spectrum which is used for selection of the calibration model according to the variation of the difference spectrum after a certain period of time.

4. The blood sugar value estimation apparatus as set forth in claim 2, wherein
when the blood sugar value estimation apparatus makes a plurality of the change of the calibration models within the period for measurement, the blood sugar value estimation apparatus calculates a first estimated blood sugar value on the basis of the calibration model which is previously used and calculates a second estimated blood sugar value on the basis of the calibration model which is newly employed,
the blood sugar value estimation apparatus is configured to make a bias correction such that the first estimated blood sugar value and the second blood sugar value are coincided with each other, and
then the blood sugar value estimation apparatus is configured to estimate the blood sugar value.

5. The blood sugar value estimation apparatus as set forth in any one of claims 1 to 4, wherein
said blood sugar value estimation apparatus is configured to make the change of the calibration model according to a development of a peak of a water in the difference spectrum.

6. The blood sugar value estimation apparatus as set forth in any one of claims 1 to 4, wherein
said blood sugar value estimation apparatus is configured to make the variation of the calibration model according to a development of a peak of a fat in the difference spectrum.

7. The blood sugar value estimation apparatus as set forth in any one of claims 1 to 4, wherein
the blood sugar value estimation apparatus is configured to make the variation of the calibration model according to a development of a peak of a water in the difference spectrum and a development of a peak of a fat in the difference spectrum.

8. The blood sugar value estimation apparatus as set forth in any one of claims 1 to 7, wherein
a plurality of the calibration models or a plurality of the datasets for creating the calibration model is created by a numerical simulation,
the numerical simulation includes a disturbance having at least one of a variation of an optical constant of water and a variation of an optical constant of fat.

9. The blood sugar value estimation apparatus as set forth in any one of claims 1 to 8, wherein
the dataset for creating the calibration model corresponding to the development of the peak of the water or the calibration model corresponding to the development of the peak of the water is created by a numerical simulation,
the numerical simulation is set to simulate a propagation of light through skin tissue,
the numerical simulation includes a disturbance which is a variation of an optical constant, the variation of the optical constant corresponds to at least a variation of an amount of water in the surface skin tissue.

10. The blood sugar value estimation apparatus as set forth in any one of claims 1 to 8, wherein
the calibration model corresponding to the development of the peak of the fat or the dataset for creating the calibration model corresponding to the development of the peak of the fat is created by the numerical simulation,
the numerical simulation is set to simulate a propagation of light through skin tissue,
the numerical simulation includes a disturbance which is a variation of an optical constant, the variation of the optical constant corresponds to at least a variation of a concentration of fat in the subcutaneous tissue.

11. The blood sugar value estimation apparatus as set forth in any one of claims 1 to 8, wherein
said calibration model corresponding to the development of the peak of the fat or the dataset for creating the calibration model corresponding to the development of the peak of the fat is created by a numerical simulation,
the numerical simulation is set to simulate a propagation of light through skin tissue,
the numerical simulation includes a disturbance which is a variation of the optical constant, the variation of the optical constant corresponds to at least a variation of scattering characteristic in surface skin.

12. The blood sugar value estimation apparatus as set forth in any one of claims 1 to 11, wherein
said blood sugar value estimation apparatus comprises a light source, a measurement probe, and a computing unit,
said light source is configured to emit light,
said measurement probe is configured to receive the light, said measurement probe is configured to apply the light, received by the measurement probe, to the living body, when the light applied to the living body is diffusely refracted in the living body, a diffusely reflected light is developed,
said measurement probe is configured to receive the diffusely reflected light,
said computing unit is configured to extract the optical spectrum, of the living body, included in the diffusely reflected light,
said computing unit comprises the calibration model creating means.

13. The blood sugar value estimation apparatus as set forth in any one of claims 1 to 12, wherein
said computing unit comprises a measurement means and a blood sugar value estimation means,
said measurement means is configured to extract an optical spectrum of the living body included in the diffusely reflected light,
said blood sugar value estimation means is configured to non-invasively calculate the blood sugar value with time on the basis of the optical spectrum, of the living body, measured by the measurement means with time and the calibration model.

14. The blood sugar value estimation apparatus as set forth in any one of claims 1 to 13, wherein
said calibration model creating means comprises a reference spectrum setting means, a measurement spectrum setting means, a difference spectrum calculating means, and a calibration model changing means,
said reference spectrum setting means is configured to measure the bio-spectrum of the person being tested to set the reference spectrum,
said measurement spectrum setting means is configured to measure the bio-spectrum in a time different from a time when the reference spectrum is measured, and set the measurement spectrum which is defined by the bio-spectrum measured in the time different from the time when the reference spectrum is measured as the measurement, said difference spectrum calculating means is configured to calculate the difference spectrum which is a difference between the measurement spectrum and the reference spectrum,
said calibration model changing means is configured to change the calibration model for calculating according to the variation of the difference spectrum.

15. The blood sugar value estimation apparatus as set forth in any one of claims 1 to 14, wherein
said measurement spectrum setting means is configured to set the measurement spectrum in present as a first measurement spectrum, said measurement spectrum setting means is configured to set the measurement spectrum of a previous time as a second measurement spectrum,
the difference spectrum calculating means is configured to calculate a first difference spectrum which is defined by a difference between the first measurement spectrum and the reference spectrum,
the difference spectrum calculating means is configured to calculate a second difference spectrum which is defined by a difference between the second measurement spectrum and the reference spectrum,
the difference spectrum calculating means is configured to calculate a variation of a difference spectrum which is defined by a difference between the first difference spectrum and the second difference spectrum,
the calibration model changing means is configured to make the change of the calibration model for calculating on the basis of the variation of the difference spectrum.

16. The blood sugar value estimation apparatus as set forth in any one of claims 1 to 14, wherein
said measurement spectrum setting means is configured to set the measurement spectrum in present as a first measurement spectrum, said measurement spectrum setting means is configured to set the measurement spectrum which is measured in a previous time from the present time as a second measurement spectrum,
the reference spectrum setting means is configured to measure the bio-spectrum of the person being tested with every a first predetermined period of time after setting the reference spectrum again to set the reference spectrum which is measured by the reference spectrum setting means,
the reference spectrum measurement means is configured to set a first reference spectrum which is defined by the reference spectrum which is measured before a second predetermined period of time from a time when said reference spectrum measurement means measures said first measurement spectrum,
said reference spectrum measurement means is configured to set a second reference spectrum which is defined by the reference spectrum which is measured by said reference spectrum measurement means in a previous time from a time when the first reference spectrum is measured,
said difference spectrum calculating means is configured to calculate said first difference spectrum which is a difference between the first measurement spectrum and the first reference spectrum,
said difference spectrum calculating means is configured to calculate a second difference spectrum which is a difference between said second measurement spectrum and said second reference spectrum,
said difference spectrum calculating means is configured to calculate a variation of the difference spectrum which is a difference between the first difference spectrum and the second difference spectrum,
said calibration model changing means is configured to change the calibration model for calculating on the basis of the variation of the difference spectrum.

17. The blood sugar value estimation apparatus as set forth in claim 16, wherein
said first reference spectrum is the reference spectrum which is measured before the first measurement spectrum is measured,
said first reference spectrum is a latest reference spectrum among the reference spectrums which are measured before the first measurement spectrum is measured.

18. The blood sugar value estimation apparatus as set forth in any one of claims 1 to 17, wherein
said blood sugar value estimation means is configured to non-invasively calculate the blood sugar value with time on the basis of a predetermined calibration model and the optical spectrum of the living body which is measured by the measurement means with time when said difference spectrum calculating means calculates no difference spectrum.

19. The blood sugar value estimation apparatus as set forth in any one of claims 1 to 18, wherein
said calibration model changing means is configured to create a predetermined calibration model from a plurality of the calibration models or a plurality of the datasets for creating the calibration model when said difference spectrum calculating means calculates no difference spectrum,
said blood sugar value estimation means is configured to non-invasively calculate the blood sugar value with time on the basis of the predetermined calibration model and the optical spectrum, of the living body, measured by the measurement means with time when the difference spectrum calculating means calculates no difference spectrum.
